# FASCICULE DE BREVET EUROPEEN

(11) **EP 0 996 530 B1**
(45) Date de publication et mention de la délivrance du brevet: **08.01.2003**
(21) Numéro de dépôt: 98939686.6
(22) Date de dépôt: 16.07.1998
(51) Int. Cl.: B29C 49/00, B29C 49/42, A61L 2/18

(54) **PROCEDE POUR LA FABRICATION DE RECIPIENTS STERILES EN MATIERE PLASTIQUE, ET INSTALLATION POUR LA MISE EN OEUVRE**
VERFAHREN ZUM HERSTELLEN VON STERILEN BEHÄLTERN AUS KUNSTOFFMATERIAL UND ANLAGE ZUR DURCHFÜHRUNG DES VERFAHRENS
METHOD FOR MAKING STERILIZED PLASTIC CONTAINERS, AND INSTALLATION THEREFOR

(30) Priorité: 18.07.1997 FR 9709335
(43) Date de publication de la demande: 03.05.2000
(73) Titulaire: SIDEL S.A., 76053 Le Havre Cédex (FR)
(72) Inventeur: MARCHAU, Bernard, F-76053 Le Havre Cedex (FR); MIE, Patrick, F-76053 Le Havre Cedex (FR); BONNEL, Christian, F-76053 Le Havre Cedex (FR); QUETEL, François, F-76053 Le Havre Cedex (FR)
(74) Mandataire: Siloret, Patrick
(86) Numéro de dépôt international: FR9801552
(87) Numéro de publication internationale: WO99003667

(56) Documents cités:
- EP-A- 0 411 769
- EP-A- 0 464 933
- WO-A-96/18541
- DE-A- 19 642 987
- US-A- 3 911 640
- US-A- 4 880 581
- PATENT ABSTRACTS OF JAPAN vol. 097, no. 002, 28 février 1997 & JP 08 282789 A (MITSUBISHI HEAVY IND LTD), 29 octobre 1996

## Description

L'invention est relative à un procédé pour la fabrication de récipients stériles en matière plastique, et à une installation pour la mise en oeuvre.

Elle s'applique tout particulièrement à la fabrication de récipients stériles à partir de préformes qui sont chauffées, pour les porter à leur température de moulage (c'est-à-dire une température à laquelle la matière plastique est ramollie), puis introduites dans un moule de finition où le récipient final est obtenu par soufflage ou étirage/soufflage de la préforme.

Elle trouve une application tout particulièrement avantageuse dans les installations en ligne ou encore dans les installations combinées (encore appelées monoblocs) pour la fabrication, le remplissage et la fermeture de récipients, dans lesquelles les récipients stériles sont remplis puis bouchés immédiatement après leur fabrication.

Une installation combinée ou monobloc est décrite dans le brevet français 74.37155 au nom de la Société des Machines pour la Transformation des Plastiques: dans ce type d'installation, la fabrication, le remplissage et la fermeture des récipients ont lieu dans une même enceinte, balayée par un flux d'air stérile.

Le brevet US 3,809,768 décrit une installation en ligne, c'est-à-dire une installation dans laquelle chaque partie, ayant une fonction particulière, est distincte des autres, et un environnement stérile est maintenu dans chacune des parties et, bien entendu, lors des transferts entre deux parties successives.

Pour obtenir des récipients stériles, diverses solutions ont été jusqu'alors envisagées.

Une solution consiste à stériliser ou décontaminer les récipients après leur fabrication, par exemple en les remplissant avec, ou en les immergeant dans, une solution de stérilisation appropriée. Cette solution pose les inconvénients suivants :
1) Tout d'abord, après remplissage ou immersion, il faut vider les récipients, éventuellement les rincer à l'aide d'une solution de rinçage, puis les assécher car le contenu final du récipient peut être dénaturé par la solution de stérilisation (ou de décontamination), ou la solution de rinçage lorsque cette dernière est utilisée.
   Cette solution est donc difficilement applicable à une installation de remplissage en ligne en raison du temps qu'elle nécessite pour sa mise en oeuvre.
2) Par ailleurs, après vidange des récipients, il faut récupérer la solution de stérilisation ou de décontamination, voire la solution de rinçage, et la (les) recycler. En effet, il n'est pas possible d'envisager de jeter la (les) solution(s) après une seule utilisation, en raison des consommations considérables que cela engendrerait. Il faut donc prévoir une installation de recyclage de chaque solution utilisée. On aboutit donc à des installations très encombrantes et coûteuses.

Par ailleurs, d'un point de vue psychologique, l'utilisateur peut se poser la question de la qualité du recyclage.

Une autre solution consiste à stériliser les préformes avant leur introduction dans la machine de fabrication de récipients. Pour ce faire, les préformes sont remplies d'une solution de stérilisation / décontamination par trempage ou remplissage, et maintenues au contact de la solution pendant un certain temps; les préformes sont ensuite vidées et les traces de solution sont retirées avant que les préformes ne soient introduites dans le four de réchauffage en vue de leur soufflage.

Cette solution présente les inconvénients suivants :
1) d'une part, le temps de contact entre la solution de stérilisation et chaque préforme doit être long (typiquement plus d'une minute avec du péroxyde d'hydrogène (H202) et encore, la qualité de la stérilisation est variable d'une préforme à l'autre), ce qui suppose une installation de stérilisation d'encombrement important pour soutenir les cadences de soufflage élevées classiquement rencontrées dans les installations de soufflage ou d'étirage / soufflage;
2) l'installation de stérilisation doit être reliée à l'installation de chauffage par un tunnel stérile;
3) la vidange des préformes, après stérilisation / décontamination implique, comme dans le cas des récipients, de recueillir la solution de nettoyage et de la recycler dans un dispositif annexe.

L'invention a pour but de remédier aux inconvénients de l'art antérieur en proposant un procédé et une installation compatible avec les cadences élevées de soufflage et qui soit d'encombrement minimum, tout en étant efficace, bien entendu.

Selon l'invention, un procédé pour obtenir un récipient stérile à partir d'une préforme en matériau thermoplastique qui est chauffée puis soufflée, alternativement étirée et soufflée, consiste à :1) appliquer d'un produit de stérilisation, activable par la chaleur, à ladite préforme, et 2) chauffer ladite préforme pour simultanément activer ledit fluide et augmenter la température de ladite préforme en vue d'atteindre sa température de moulage, et 3) souffler, alternativement étirer et souffler la préforme, pour obtenir le récipent final.

Ainsi, en chauffant la préforme pour à la fois activer le produit de stérilisation et augmenter la température de la préforme afin d'atteindre sa température de soufflage, on réalise en une seule opération la stérilisation et le ramollissement du matériau constitutif de la préforme en vue de son soufflage. On raccourcit donc le cycle global.

On s'affranchit ainsi de la nécessité de prévoir une installation spéciale pour la stérilisation puisqu'on utilise une installation commune pour le chauffage aux fins d'activation du produit de stérilisation et du ramollissement de la préforme. Il suffit de prévoir des moyens pour appliquer le produit dans les préformes.

Accessoirement, on économise de l'énergie.

La sécurité de l'opération de stérilisation est totale, puisque les récipients sont soufflés immédiatement après que les préformes aient été stérilisées : or les préformes sont stérilisées en étant chauffées et la température à laquelle elles sont introduites dans les moules, puis soufflées, est telle que de nouveaux germes ne peuvent pas se développer.

Avec les procédés de l'art antérieur, selon lesquels les préformes sont stérilisées, asséchées et transférées vers le four, il existe le risque d'une pollution des préformes durant le transfert, par exemple si le tunnel stérile connaît une défaillance. Ce type de risque est supprimé ici.

Par ailleurs, avec le procédé de l'invention, l'activation du produit par la chaleur a pour effet de maintenir stériles les organes en contact avec les préformes au moins dans la zone de chauffage. Il n'est donc pas nécessaire de prévoir un flux d'air stérile dans cette zone.

Selon une autre caractéristique, l'application du produit de stérilisation est précédée d'une phase de suppression de l'électricité statique dans la préforme.

Ainsi, les particules et germes sont plus facilement traités par la suite, car ils se décollent de la paroi de la préforme.

Selon une autre caractéristique, le produit est un fluide et son application s'effectue par mouillage de la préforme.

De façon surprenante, on a en effet constaté qu'il n'est pas nécessaire d'introduire de grandes quantités de produit pour stériliser des préformes, et qu'un simple mouillage suffit donc.

Dans une mise en oeuvre, le mouillage s'effectue au moment de l'entrée de la préforme dans l'installation, en amont des moyens de chauffage, en pulvérisant la quantité nécessaire de fluide dans la préforme, de façon à mouiller au moins toute sa surface intérieure.

Dans une autre mise en oeuvre, le mouillage s'effectue en remplissant la préforme et en la vidant ensuite sans l'assécher, avant de la faire entrer dans les moyens de chauffage.

Cette autre mise en oeuvre présente l'avantage, par rapport au procédé de l'art antérieur sus-mentionné, de nécessiter une installation de recyclage de moindre performance puisque l'essentiel de la décontamination a lieu lors du chauffage. Le produit issu de la vidange n'est donc que peu contaminé.

Selon une autre caractéristique, la quantité de produit placée dans la préforme est telle que non seulement la préforme sort stérile des moyens de chauffage, mais encore la totalité de produit s'évapore lors du chauffage, ce qui évite de perturber la phase de moulage du récipient final. En effet, des traces de produit pourraient être néfastes à l'obtention d'un moulage correct.

Dans une mise en oeuvre, le soufflage final s'effectue à l'aide d'air suffisamment filtré pour ne pas réintroduire de particules indésirables.

Il est donc possible d'utiliser une installation de soufflage classique avec des filtres appropriés.

Dans une autre mise en oeuvre, le soufflage final s'effectue à l'aide à l'aide d'un fluide stérile, tel de l'air stérile, de sorte que le récipient peut être immédiatement rempli et/ou bouché après soufflage.

D'autres caractéristiques et avantages de la présente invention apparaîtront à la lecture de la description qui suit, faite en regard des figures annexées sur lesquelles :
- la figure 1 est un schéma de principe de l'invention;
- la figure 2 est un schéma de principe illustrant une première variante de l'invention;
- les figures 3 et 4 sont des schémas de principe d'une partie d'une seconde variante de l'invention;
- la figure 5 est un schéma plus détaillé d'une installation à laquelle l'invention peut s'appliquer;
- les figures 6 à 8 illustrent, schématiquement, des variantes perfectionnées.

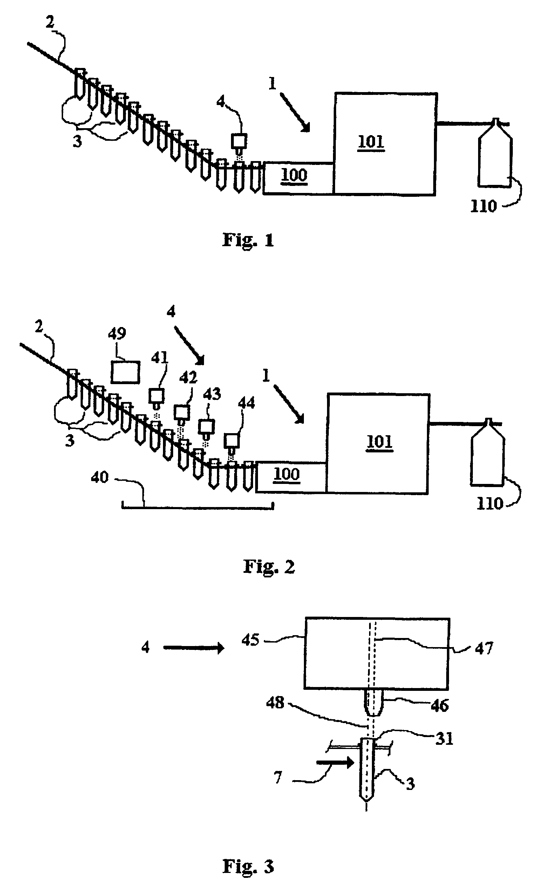

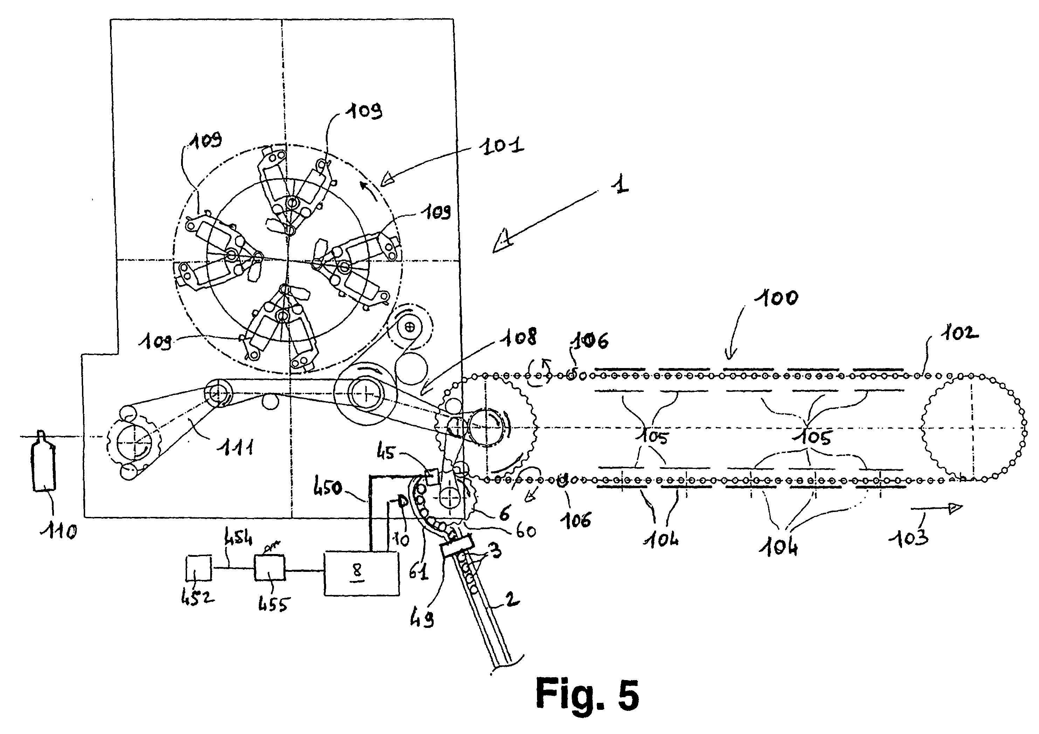

Sur la figure 1, on a représenté le schéma de principe d'une installation pour la mise en oeuvre du procédé de l'invention.

L'installation, plus généralement désignée sous la référence 1, est reliée par un dispositif 2 d'amenée des préformes 3 à une source d'alimentation en préformes 3 non représentée. Cette source peut consister de façon connue en une trémie d'alimentation et le dispositif 2 est alors, par exemple, un rail d'alimentation. Alternativement, et sans que cela soit limitatif, la source peut consister en une presse d'injection des préformes et le dispositif 2 est un convoyeur d'amenée des préformes.

L'installation 1 comporte, de façon connue, des moyens 100 de chauffage tels qu'un four pour porter les préformes à leur température de soufflage (typiquement de l'ordre de 100°C à 150°C lorsque le matériau constitutif des préformes 3 est le polyéthylènetéréphtalate (PET). Les préformes 3 amenées par le dispositif 2 d'amenée sont introduites dans le four 100 où elles sont conditionnées thermiquement, avant d'être transférées dans le dispositif 101 de soufflage, tel qu'un carrousel ou roue de soufflage, des récipients 110.

Conformément à l'invention, des moyens 4 sont prévus en amont des moyens 100 de chauffage pour appliquer du produit de stérilisation au moins à l'intérieur des préformes 3.

De préférence, le produit de stérilisation est une solution chimique activable par la chaleur, typiquement une solution oxydante, telle qu'une solution à base d'acide péracétique ou, de préférence, de péroxyde d'hydrogène (H202), ou toute autre solution appropriée.

Dans une mise en oeuvre (figure 2), les moyens 4 sont constitués par une série de buses 41;... 44 coulant en permanence et remplissant les préformes 3 au défilé, par exemple lorsqu'elles se trouvent encore sur le dispositif 2 d'amenée. Dans ce cas, on prévoit des moyens, tel un bac 40 pour récupérer la solution en excès, débordant des préformes.

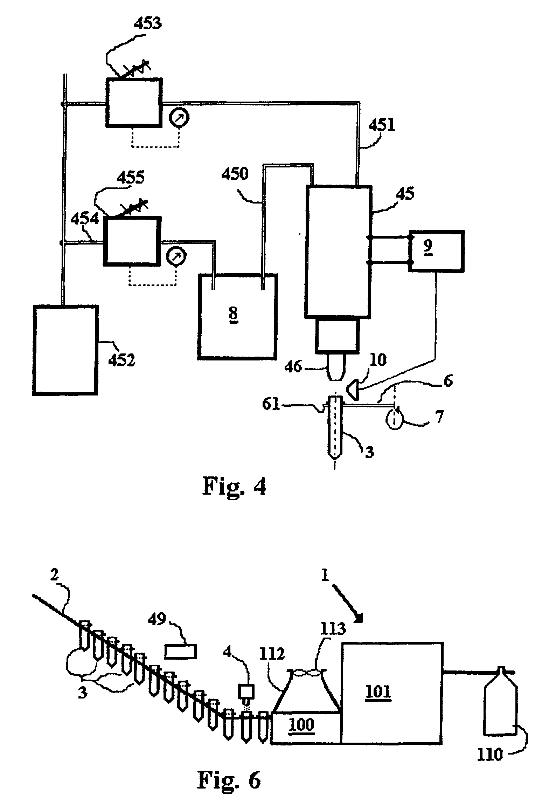

Une telle mise en oeuvre nécessite de vider les préformes en les retournant et de récupérer et recycler le produit de vidange des préformes et du bac 40. La vidange des préformes peut être effectuée de façon avantageuse à l'entrée du four dans une zone 5, visible sur la figure 4, où les préformes sont positionnées col en bas.

Néanmoins, cette mise en oeuvre n'est pas la plus pratique, car elle nécessite une récupération du produit en excès lors du remplissage et issu de la vidange, de même que son recyclage. Il faut cependant noter que, lors du remplissage, le produit se pollue peu car il n'a pas réellement le temps de commencer à agir, l'activation se faisant ultérieurement, par chauffage. Donc, le degré de recyclage est de moindre importance par rapport à ce qui est nécessité avec le procédé sus-mentionné de l'art antérieur de stérilisation des préformes en amont de l'installation.

C'est pourquoi, de préférence (figure 3), les moyens 4 sont constitués par un pulvérisateur 45 automatique tel, par exemple, un pulvérisateur à commande électrique disposé soit en regard du dispositif 2 d'amenée des préformes, soit un peu plus loin entre le dispositif d'amenée et le four 100, par exemple en regard d'un organe intermédiaire entre le dispositif 2 et le four 10, tel que la roue 6 de chargement des préformes présente dans les machines de la demanderesse et visible sur la figure 5.

Le pulvérisateur permet de mouiller l'intérieur des préformes avec la seule quantité nécessaire à leur stérilisation, et le produit s'évapore ensuite sous l'effet de la chaleur.

On a constaté, à titre d'exemple, que des pulvérisations de 0,01 g. à 0,07 g. de péroxyde d'hydrogène (H202) dans des préformes destinées à la fabrication de bouteilles de 1 litre permettent d'obtenir d'excellents résultats.

Ceci correspond, pour des préformes standard, à une quantité nécessaire inférieure au centième du volume total des préformes, en fait à des volumes compris sensiblement entre le cinq centième et le trois millième du volume total des préformes.

Au-dessus du centième, des traces de produit peuvent subsister en sortie des moyens 100 de chauffage, ce qui risque de perturber le soufflage.

A cet effet, le pulvérisateur, d'une part, est associé à un capteur, non représenté sur cette figure, mais visible 10 sur la figure 4, pour détecter l'arrivée de chaque préforme 3 et, d'autre part, est réglé pour envoyer la quantité de produit nécessaire à partir du moment de la détection.

Par exemple, à supposer que les préformes se déplacent dans le sens illustré par la flèche 7 sur la figure 3, le pulvérisateur est réglé pour que la pulvérisation commence lorsque le buvant 31 d'une préforme 3 arrive en regard de l'ensemble 46 de pulvérisation (trait interrompu 47 sur la figure 3) et cesse lorsque l'ensemble 46 est approximativement en regard de l'axe central 48 de la préforme.

En agissant ainsi, toute la paroi intérieure et le fond des préformes se trouvent assez mouillés pour permettre une stérilisation efficace lorsque le produit est activé par la chaleur régnant dans le four.

On met ici en évidence l'avantage indéniable que présente l'invention en termes de consommation de produit : avec du péroxyde d'hydrogène, dont la masse volumique est proche de celle de l'eau, il suffit d'environ 30 grammes de produit pour traiter 1000 préformes destinées à des bouteilles d'un litre. Il en faudrait 30 kilogrammes, donc 30 litres pour traiter 1000 préformes en les remplissant à ras bord, et 1000 litres pour traiter 1000 bouteilles en les remplissant à ras bord.

Dans une mise en oeuvre, le pulvérisateur 45 est constitué par un pistolet automatique basse pression, connu en soi, dans lequel la pulvérisation est obtenue par un ensemble 46 composé d'une buse d'air et d'une buse de liquide. On utilise par exemple le pistolet de marque "Autojet" vendu sous la référence 28JJ AU, par la société SPRAYING SYSTEMS Co et équipé d'un ensemble 46 de projection circulaire, constitué par une buse de liquide et par une buse d'air.

Le montage d'un tel pistolet peut être effectué conformément à la figure 4.

Le pistolet 45 est relié à un réservoir 8 de liquide de stérilisation par l'intermédiaire d'un tuyau 450 qui met en communication le réservoir et la buse de liquide, non référencée, de l'ensemble 46 de pulvérisation.

Un tuyau 451 d'arrivée d'air met en communication permanente la buse d'air, non référencée et une source 452 d'alimentation en air comprimé basse pression (typiquement entre 5 et 9 bars, mais cette plage de valeurs peut varier).

Un détendeur 453 est intercalé entre la source 452 d'air basse pression et le tuyau 451 d'arrivée d'air pour réguler la pression.

Le réservoir 8 est soumis à une surpression interne (typiquement entre 0 et 7 bars). Dans l'exemple illustré par la figure 4, la surpression est obtenue en dérivant 454 l'air issu de la source 452 vers un détendeur 455 dont la sortie est reliée au réservoir 8, afin d'obtenir la surpression désirée dans ce dernier.

Un dispositif 9 à commande électrique, relié à un capteur 10 de détection du passage des préformes 3, envoie des ordres à un circuit électromagnétique contenu dans le corps du pistolet pour déplacer le pointeau de fermeture / ouverture du passage de liquide, lui-aussi contenu dans le corps du pistolet.

De préférence, comme illustré sur la figure 5, on prévoit en outre des moyens 49 pour décharger les préformes de l'électricité statique qu'elles contiennent. Ces moyens sont en amont du pulvérisateur et sont, dans un mode de réalisation préféré, constitués par un générateur d'air ionisé qui envoie (injecte) un flux d'air ionisé à l'intérieur des préformes. Le flux d'air favorise le décollement des particules et l'ionisation empêche que les particules adhèrent à la paroi des préformes.

Comme illustré par la figure 3, l'envoi d'air ionisé a lieu par exemple lorsque les préformes sont encore sur le dispositif 2 d'amenée (rail ou convoyeur), peu avant le pulvérisateur 45.

Un tel dispositif 49 a été également représenté sur la figure 2. Il peut être utilisé dans tout mode de mise en oeuvre de l'invention.

Sur la figure 5 est illustrée schématiquement en outre la façon dont l'invention peut être adaptée à des équipements de soufflage connus en soi, tels les équipements fabriqués par la demanderesse.

Les préformes 3 sont amenées dans l'installation par des moyens tels une glissière ou rail 2. Elles sont prises en charge sous leur collerette entre une roue 6 à encoches 60 et un guide 61 semi-circulaire.

Le pulvérisateur 45 est placé au-dessus du trajet des préformes 3, par exemple dans la zone où elles sont portées par les encoches 60 de la roue 6 et le guide 61, et le capteur 10 est alors placé en regard de cette zone pour détecter l'arrivée d'une préforme 3 sous le pulvérisateur.

Pour ne pas surcharger cette figure, les circuits de mise en surpression du réservoir 8, et plus généralement les circuits fluidiques représentés sur la figure 4 n'ont pas été tous représentés.

Après leur passage sur la roue 6, les préformes sont saisies une par une par une chaîne 102 de tournettes, connue en soi, les transportant dans le four 100. Les préformes 3, saisies par leur col, sont entraînées dans le sens illustré par la flèche 103 et passent devant des moyens de chauffage (lampes 104 et réflecteurs 105). Par ailleurs, les préformes, après leur introduction dans le four, sont retournées pour être mises col en bas, puis sont mises en rotation sur elles-mêmes (flèche 106) pendant au moins leur passage devant les moyens de chauffage 104, 105.

A la sortie du four, les préformes sont remises col en haut, avant d'être saisies par des organes de transfert 108 pour les diriger vers les moules 109 de finition du carrousel 101 de soufflage. Les récipients achevés 110 sont ensuite évacués par des moyens 111 appropriés.

On conçoit donc que l'invention est très facile à mettre en oeuvre sur des installations de soufflage connues et n'impose que peu de modifications.

Il suffit de disposer le pulvérisateur 45 et ses organes annexes (réservoir 8, capteur 10) et de relier ses circuits d'air aux circuits d'air existant dans l'installation.

Il peut être cependant nécessaire de choisir des matériaux inoxydables qui résistent aux produits de stérilisation, notamment pour le four 100 et les autres éléments constitutifs de l'installation.

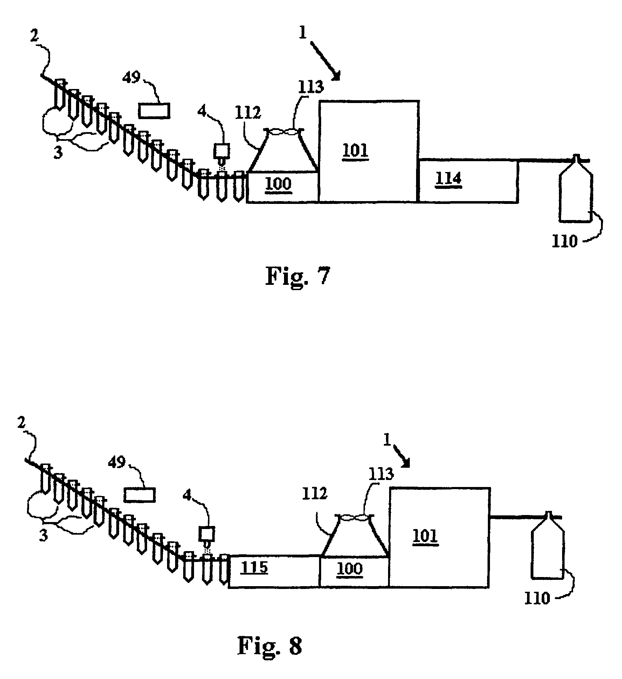

D'autres adaptations peuvent être souhaitables qui sont illustrées sur les figures 6 et 7.

D'une part, il peut être préférable de prévoir un ensemble d'aspiration et d'évacuation des vapeurs résiduelles de produit de stérilisation, constitué par exemple par une hotte 112 et une turbine 113 d'aspiration, disposé au-dessus du four 100, sur ces figures 6 et 7.

Dans le mode de mise en oeuvre de la figure 6, l'ensemble 101 de soufflage utilise de l'air stérile pour former les récipients. En plus du pulvérisateur 45, de ses moyens annexes associés, et de l'ensemble d'aspiration 112, 113, il est préférable de prévoir une surpression avec de l'air stérile dans l'installation pour éviter que des particules septiques ne pénètrent à l'intérieur.

Dans le mode de mise en oeuvre de la figure 7, l'ensemble 101 de soufflage utilise, non pas de l'air stérile, mais de l'air filtré de façon adéquate, et on prévoit dans ce cas, à la sortie de cet ensemble 101, à titre de précaution, un dispositif annexe 114 pour supprimer et/ou détruire les éventuelles particules septiques réintroduites lors du soufflage. Le dispositif annexe 114 est par exemple constitué par une rinceuse, utilisant un produit de décontamination approprié, et il peut être complété par une sécheuse des récipients 110, notamment pour éviter que le produit de décontamination ne modifie le goût du produit de remplissage.

Bien entendu, tout autre dispositif équivalent permettant de détruire des particules contaminantes susceptibles d'être présentes en faible quantité peut être utilisé.

Dans une variante, illustrée par la figure 8, et applicable aux modes de réalisation de la figure 5 (à condition d'en modifier légèrement la configuration) et des figures 6 ou 7, les préformes 3, après mouillage par remplissage 41;...;44, puis vidange, ou simple pulvérisation 45, sont introduites dans un dispositif 115 de chauffage préalable pour démarrer l'activation du produit de stérilisation et commencer leur montée en température en vue de leur soufflage.

Ensuite, elles sont transférées dans le four 100, situé en aval du dispositif 115, dans lequel elles subissent un profil de chauffe approprié en vue de leur soufflage et dans lequel la décontamination continue et l'évaporataion du produit s'effectue, du fait de la chaleur y régnant. La disposition relative du dispositif 115 préalable et du four 100 est telle que le transfert entre ces deux organes s'effectue sans refroidissement notable des préformes pour éviter une désactivation précoce du produit et une consommation d'énergie excessive.

En effet, si les préformes étaient refroidies, il n'y aurait aucun bénéfice sur le plan énergétique et on perdrait alors l'un des avantages de l'invention.

Une installation de fabrication de récipients stériles conforme à l'invention peut être directement connectée à une installation de remplissage et/ou de bouchage pour constituer soit une installation monobloc, soit une installation en ligne. Dans ce dernier cas, il faut prévoir un tunnel stérile entre la sortie des récipients de la souffleuse 100 et l'entrée de la remplisseuse et/ou de la boucheuse.

Bien entendu, l'invention n'est pas limitée aux modes de réalisation et d'application qui ont été plus particulièrement envisagés : elle en embrasse, au contraire, toutes les contraintes.

## Revendications

1. Procédé pour obtenir un récipient (110) stérile, à partir d'une préforme (3) en matériau thermoplastique qui est chauffée (100) puis moulée (101) par soufflage, alternativement par étirage et soufflage, consistant à :
1) appliquer (4;41;...44,45) un produit de stérilisation, activable par la chaleur, à ladite préforme (3) et
2) chauffer la préforme pour simultanément activer ledit produit de stérilisation et augmenter la température de la préforme en vue d'atteindre sa température de moulage, et
3) mouler (101) ladite préforme par soufflage, alternativement étirage et soufflage, pour obtenir le récipient (110) final.

2. Procédé selon la revendication 1, **caractérisé en ce que** l'application du produit de stérilisation est précédée d'une phase de suppression de l'électricité statique présente dans la préforme (3).

3. Procédé selon la revendication 2, **caractérisé en ce que** la suppression de l'électricité statique présente dans la préforme (3) est obtenue par injection (49) d'air ionisé dans la préforme (3).

4. Procédé selon l'une des revendications 1 à 3, **caractérisé en ce que** le produit de stérilisation est un fluide et que son application (4) sur la préforme s'effectue par mouillage d'au moins les parois intérieures de ladite préforme (3) avec un volume de fluide suffisant pour la stérilisation.

5. Procédé selon la revendication 4, **caractérisé en ce que** le mouillage (4) s'effectue par remplissage (41;...44) puis vidange de la préforme, sans assèchement de celle-ci, pour qu'il subsiste un volume de fluide suffisant pour la stérilisation.

6. Procédé selon la revendication 4, **caractérisé en ce que** le mouillage (4) s'effectue en pulvérisant (45, 46) dans la préforme le volume de fluide suffisant pour la stérilisation.

7. Procédé selon l'une des revendications 2 à 6, **caractérisé en ce que** le produit de stérilisation est évaporable et le volume de produit de stérilisation appliqué est tel qu'il est totalement évaporé à l'issue du chauffage avant moulage.

8. Procédé selon l'une des revendications 2 à 7, **caractérisé en ce que** le produit de stérilisation est un oxydant.

9. Procédé selon la revendication 8, **caractérisé en ce que** le produit de stérilisation est à base de péroxyde d'hydrogène.

10. Procédé selon la revendication 8, **caractérisé en ce que** le produit de stérilisation est à base d'acide péracétique.

11. Procédé selon l'une des revendications 4 à 10 **caractérisé en ce que** le volume de fluide est inférieur au centième du volume total de la préforme.

12. Procédé selon la revendication 11, **caractérisé en ce que** le volume de fluide est compris entre le cinq centième et le trois millième de volume total de la préforme.

13. Procédé selon l'une des revendications 1 à 12, **caractérisé en ce que** le soufflage du récipient (110) est effectué à l'aide d'un fluide stérile, tel de l'air stérile, de sorte que le récipient peut être immédiatement rempli et/ou bouché après soufflage.

14. Procédé selon l'une des revendications 1 à 12, **caractérisé en ce que** le soufflage du récipient s'effectue à l'aide d'air filtré et est complété, à titre de précaution, par une opération (114) de suppression et/ou destruction des éventuelles particules septiques réintroduites lors du soufflage, afin de permettre un éventuel remplissage et/ou bouchage subséquent.

15. Procédé selon la revendication 14, **caractérisé en ce que** l'opération (114) de suppression et/ou destruction des éventuelles particules septiques réintroduites lors du soufflage est constituée par un rinçage à l'aide d'un produit stérilisant approprié, tel qu'une solution de liquide stérilisant; et une vidange du récipient.

16. Procédé selon la revendication 15, **caractérisé en ce que** la vidange est suivie d'un séchage du récipient.

17. Procédé selon l'une des revendications 1 à 16, **caractérisé en ce que** le chauffage de 1a préforme s'effectue en deux étapes, une première (115) pour activer l'action du produit de stérilisation et commencer la montée en température de la préforme, et une seconde (100) pour continuer l'action du produit de stérilisation, l'évaporer et conférer un profil de chauffe déterminé à la préforme en vue de son moulage.

18. Installation pour la mise en oeuvre du procédé selon l'une des revendications 1 à 17, comportant des moyens (100, 115) de chauffage des préformes et des moyens de moulage (101; 109) par soufflage, alternativement par étirage et soufflage, de récipients (110) à partir des préformes (3) chauffées, et comportant, en amont des moyens (100) de chauffage, des moyens (4; 41; ... 44; 45) pour appliquer du produit de stérilisation, activable par la chaleur, aux dites préformes (3) et des moyens pour transférer les préformes avec le produit de stérilisation dans les moyens de chauffage afin d'activer le produit de stérilisation et porter les préformes à leur température de moulage.

19. Installation selon la revendication 18, **caractérisée en ce que** le produit de stérilisation est un liquide et **en ce que** les moyens pour appliquer le produit de stérilisation aux préformes sont agencés pour mouiller les préformes.

20. Installation selon la revendication 19, **caractérisée en ce que** les moyens pour mouiller les préformes sont constitués d'une part par une série de buses (41; ...; 44) situées au-dessus du trajet des préformes en amont de l'installation, afin de remplir les préformes lorsqu'elles passent au défilé en regard de ces buses, et d'autre part par des moyens (5) pour retourner et vider les préformes sans les assécher.

21. Installation selon la revendication 20, **caractérisé en ce que** les moyens pour retourner les préformes sont constitués par des moyens (5) de retournement afin de mettre les préformes col en bas dans les moyens (100) de chauffage.

22. Installation selon la revendication 19, **caractérisée en ce que** les moyens pour mouiller les préformes sont constitués par un pulvérisateur (45), tel un pistolet automatique à commande électrique basse pression.

23. Installation selon la revendication 22, **caractérisé en ce que** le pistolet est alimenté en air (451) et en liquide de pulvérisation (450) et le passage du liquide est commandé par un organe de commande (9) activé par un capteur (10) de position des préformes.

24. Installation selon l'une des revendications 18 à 23, **caractérisée en ce que** les moyens (100) de chauffage pour activer le produit de stérilisation et porter les préformes à leur température de moulage sont constituées par le four (100) pour le conditionnement thermique des préformes en vue de leur moulage.

25. Installation selon l'une des revendications 18 à 23, **caractérisée en ce que** les moyens de chauffage sont constitués d'une part par une enceinte (115) de chauffage en aval des moyens (4) pour appliquer le produit de stérilisation et d'autre part par le four (100) de chauffage des préformes pour leur conditionnement thermique en vue du moulage des récipients finaux (110), ledit four (100) est en aval de l'enceinte (115), et des moyens de transfert sont prévus entre l'enceinte (115) et le four (100) et sont agencés pour que les préformes ne soient pas refroidies lors du passage de l'enceinte au four.

26. Installation selon l'une des revendications 18 à 25, **caractérisée en ce qu'**elle comporte des moyens pour souffler de l'air stérile dans les préformes en vue de leur moulage en récipients (110) finaux.

27. Installation selon l'une des revendications 18 à 25, **caractérisée en ce qu'**elle comporte des moyens pour souffler de l'air filtré dans les préformes en vue de leur moulage en récipients (110) finaux.

28. Installation selon 1a revendication 27, **caractérisée en ce qu'**elle comporte des moyens (114) pour effectuer une opération de suppression et/ou destruction des éventuelles particules septiques réintroduites lors du soufflage, telle une opération de rinçage et vidange, des récipients (110).

29. Installation selon l'une des revendications 18 à 28, **caractérisée en ce qu'**elle comporte, en amont des moyens (4; 41 ... 44; 45) pour appliquer le produit de stérilisation, des moyens (49) pour supprimer l'électricité statique présente dans les préformes.

30. Installation selon la revendication 29, **caractérisée en ce que** les moyens pour supprimer l'électricité statique sont constitués par un dispositif (49) d'injection d'air ionisé dans les préformes.

31. Installation selon l'une des revendications 18 à 30, **caractérisée en ce qu'**elle est reliée à une installation de remplissage et/ou de bouchage des récipients (110).

## Patentansprüche

1. Verfahren zum Erhalten eines sterilen Behälters (110) ausgehend von einer Vorform (3) aus thermoplastischem Material, die erwärmt (100) und dann durch Blasformen, alternativ durch Streck- und Blasformen, geformt (101) wird, das aus folgenden Schritten besteht:
1) Anwenden (4; 41; .... 44, 45) eines Sterilisierprodukts, das durch Wärme aktivierbar ist, auf die Vorform (3) und
2) Erwärmen der Vorform, um gleichzeitig das Sterilisierprodukt zu aktivieren und die Temperatur der Vorform hinsichtlich des Erreichens ihrer Formungstemperatur zu erhöhen und
3) Formen (101) der Vorform durch Blasformen, alternativ durch Streck- und Blasformen, um den endgültigen Behälter (110) zu erhalten.

2. Verfahren nach Anspruch 1, **dadurch gekennzeichnet, dass** der Anwendung des Sterilisierprodukts eine Phase der Beseitigung der in der Vorform (3) vorhandenen statischen Elektrizität vorausgeht.

3. Verfahren nach Anspruch 2, **dadurch gekennzeichnet, dass** die Beseitigung der in der Vorform (3) vorhandenen statischen Elektrizität durch Einblasen (49) ionisierter Luft in die Vorform (3) erhalten wird.

4. Verfahren nach einem der Ansprüche 1 bis 3, **dadurch gekennzeichnet, dass** das Sterilisierprodukt ein Fluid ist und dass seine Anwendung (4) auf die Vorform durch Formen zumindest der Innenwände der Vorform (3) mit einem zur Sterilisierung ausreichenden Fluidvolumen erfolgt.

5. Verfahren nach Anspruch 4, **dadurch gekennzeichnet, dass** das Formen (4) durch Füllen (41; .... 44) und dann Entleerung der Vorform ohne deren Trockenlegung erfolgt, damit ein für die Sterilisierung ausreichendes Fluidvolumen übrigbleibt.

6. Verfahren nach Anspruch 4, **dadurch gekennzeichnet, dass** das Formen (4) durch Versprühen (45, 46) des zur Sterilisierung ausreichenden Fluidvolumens in der Vorform erfolgt.

7. Verfahren nach einem der Ansprüche 2 bis 6, **dadurch gekennzeichnet, dass** das Sterilisierprodukt verdampfbar ist und das Volumen des angewandten Sterilisierprodukts derart ist, dass es am Ende der Erwärmung vor dem Formen vollständig verdampft ist.

8. Verfahren nach einem der Ansprüche 2 bis 7, **dadurch gekennzeichnet, dass** das Sterilisierprodukt ein Oxidiermittel ist.

9. Verfahren nach Anspruch 8, **dadurch gekennzeichnet, dass** das Sterilisierprodukt auf Wasserstoffperoxidbasis beruht.

10. Verfahren nach Anspruch 8, **dadurch gekennzeichnet, dass** das Sterilisierprodukt auf Peressigsäurebasis beruht.

11. Verfahren nach einem der Ansprüche 4 bis 10, **dadurch gekennzeichnet, dass** das Fluidvolumen kleiner als ein Hundertstel des Gesamtvolumens der Vorform ist.

12. Verfahren nach Anspruch 11, **dadurch gekennzeichnet, dass** das Fluidvolumen zwischen fünf Hundertstel und drei Tausendstel des Gesamtvolumens der Vorform liegt.

13. Verfahren nach einem der Ansprüche 1 bis 12, **dadurch gekennzeichnet, dass** das Blasformen des Behälters (110) mittels eines sterilen Fluids durchgeführt wird, wie beispielsweise steriler Luft, so dass der Behälter sofort nach dem Blasformen gefüllt und/oder verschlossen werden kann.

14. Verfahren nach einem der Ansprüche 1 bis 12, **dadurch gekennzeichnet, dass** das Blasformen des Behälters mittels gefilterter Luft erfolgt und vorsichtshalber durch einen Vorgang (114) der Beseitigung und/oder der Vertilgung eventueller beim Blasformen eingeführter septischer Teilchen abgeschlossen wird, um eine nachfolgende eventuelle Füllung und/oder Verschließung zu gestatten.

15. Verfahren nach Anspruch 14, **dadurch gekennzeichnet, dass** der Vorgang (114) der Beseitigung und/oder der Vertilgung eventueller beim Blasformen eingeführter septischer Teilchen aus einer Spülung mittels eines geeigneten Sterilisierprodukts, wie beispielsweise einer Sterilisierflüssigkeitslösung, und einer Entleerung des Behälters besteht.

16. Verfahren nach Anspruch 15, **dadurch gekennzeichnet, dass** der Entleerung eine Trocknung des Behälters folgt.

17. Verfahren nach einem der Ansprüche 1 bis 16, **dadurch gekennzeichnet, dass** das Erwärmen der Vorform in zwei Stufen erfolgt, einer ersten (115), um die Wirkung des Sterilisierprodukts zu aktivieren und den Temperaturanstieg der Vorform einzuleiten, und einer zweiten (100), um die Einwirkung des Sterilisierprodukts fortzusetzen, es zu verdampfen und der Vorform hinsichtlich ihres Formens ein bestimmtes Erwärmungsprofil zu vermitteln.

18. Einrichtung zur Durchführung des Verfahrens nach einem der Ansprüche 1 bis 17 mit Mitteln (100, 115) zum Erwärmen der Vorformen und Mitteln zum Formen (101; 109) durch Blasformen, alternativ durch Streck- und Blasformen, von Behältern (110) ausgehend von erwärmten Vorformen (3), und mit Mitteln (4; 41; ... 44; 45) stromauf von den Erwärmungsmitteln (100), um das Sterilisierprodukt, das durch Wärme aktivierbar ist, auf die Vorformen (3) anzuwenden, und Mitteln, um die Vorformen mit dem Sterilisierprodukt in die Mittel zum Erwärmen zu übertragen, um das Sterilisierprodukt zu aktivieren und die Vorformen auf ihre Formungstemperatur zu bringen.

19. Einrichtung nach Anspruch 18, **dadurch gekennzeichnet, dass** das Sterilisierprodukt eine Flüssigkeit ist und dass die Mittel zum Anwenden des Sterilisierprodukts auf die Vorformen dafür ausgelegt sind, die Vorformen zu benetzen.

20. Einrichtung nach Anspruch 19, **dadurch gekennzeichnet, dass** die Mittel zum Bentzen der Vorformen einerseits aus einer Reihe von Düsen (41; ....; 44), die über der Bahn der Vorformen stromauf von der Einrichtung liegen, um die Vorformen zu füllen, wenn sie gegenüber von diesen Düsen vorbeilaufen, und andererseits aus Mitteln (5) bestehen, um die Vorformen zu drehen und zu leeren, ohne sie zu trocknen.

21. Einrichtung nach Anspruch 20, **dadurch gekennzeichnet, dass** die Mittel zum Umdrehen der Vorformen aus Umdrehmitteln (5) bestehen, um die Vorformen mit dem Kopf nach unten in die Erwärmungsmittel (100) zu setzen.

22. Einrichtung nach Anspruch 19, **dadurch gekennzeichnet, dass** die Mittel zur Benetzung der Vorformen aus einem Sprühgerät (45) bestehen, wie beispielsweise einer elektrisch betätigten, automatischen Niederdruckpistole.

23. Einrichtung nach Anspruch 22, **dadurch gekennzeichnet, dass** die Pistole mit Luft (451) und Sprühflüssigkeit (450) versorgt wird und der Durchtritt der Flüssigkeit durch ein Betätigungsorgan (9) betätigt wird, das durch einen Positionssensor (10) für die Vorformen aktiviert wird.

24. Einrichtung nach einem der Ansprüche 18 bis 23, **dadurch gekennzeichnet, dass** die Erwärmungsmittel (100) zur Aktivierung des Sterilisierprodukts und zur Erwärmung der Vorformen auf ihre Formungstemperatur aus dem Ofen (100) zur Konditionierung der Vorformen im Hinblick auf ihre Formung bestehen.

25. Einrichtung nach einem der Ansprüche 18 bis 23, **dadurch gekennzeichnet, dass** die Erwärmungsmittel einerseits aus einer Umfassung (115) zum Erwärmen stromab von den Mitteln (4) zum Anwenden des Sterilisierprodukts und andererseits aus dem Ofen (100) zur Erwärmung der Vorformen für ihre thermische Konditionierung im Hinblick auf das Formen der endgültigen Behälter (110) bestehen, wobei der Ofen (100) stromauf von der Umfassung (115) liegt, und Übertragungsmittel zwischen der Umfassung (115) und dem Ofen (100) vorgesehen sind und dafür ausgelegt sind, dass die Vorformen während des Übergangs von der Umfassung zum Ofen nicht gekühlt werden.

26. Einrichtung nach einem der Ansprüche 18 bis 25, **dadurch gekennzeichnet, dass** sie Mittel zum Einblasen steriler Luft in die Vorformen im Hinblick auf ihre Formung in endgültige Behälter (110) umfasst.

27. Einrichtung nach einem der Ansprüche 18 bis 25, **dadurch gekennzeichnet, dass** sie Mittel zum Einblasen gefilterter Luft in die Vorformen im Hinblick auf ihre Formung in endgültige Behälter (110) umfasst.

28. Einrichtung nach Anspruch 27, **dadurch gekennzeichnet, dass** sie Mittel (114) umfasst, um einen Vorgang der Beseitigung und/oder Vertilgung eventueller bei dem Blasformen eingeführter septischer Teilchen durchzuführen, wie beispielsweise einen Vorgang der Spülung und Entleerung der Behälter (110).

29. Einrichtung nach einem der Ansprüche 18 bis 28, **dadurch gekennzeichnet, dass** sie stromauf von den Mitteln (4; 41 ... 44; 45) zum Anwenden des Sterilisierprodukts, Mittel zum Beseitigen der in den Vorformen vorhandenen statischen Elektrizität umfasst.

30. Einrichtung nach Anspruch 29, **dadurch gekennzeichnet, dass** die Mittel zum Beseitigen statischer Elektrizität aus einer Vorrichtung (49) zum Einblasen isonisierter Luft in die Vorformen bestehen.

31. Einrichtung nach einem der Ansprüche 18 bis 30, **dadurch gekennzeichnet, dass** sie mit einer Einrichtung zum Füllen und/oder Verschließen der Behälter (110) verbunden ist.

## Claims

1. Process for obtaining a sterile container (110) from a thermoplastic preform (3) which is heated (100) and then moulded (101) by blow moulding or alternatively by stretch-blow moulding, consisting in:
1) applying (4; 41; ... 44, 45) a heat-activatable sterilization product to the said preform (3);
2) heating the preform in order simultaneously to activate the said sterilization product and increase the temperature of the preform so that it reaches its moulding temperature; and
3) moulding (101) the said preform by blow moulding, or alternatively by stretch-blow moulding, in order to obtain the final container (110).

2. Process according to Claim 1, **characterized in that** the application of the sterilization product is preceded by a phase of eliminating the static electricity present in the preform (3).

3. Process according to Claim 2, **characterized in that** the static electricity present in the preform (3) is eliminated by injecting (49) ionized air into the preform (3).

4. Process according to one of Claims 1 to 3, **characterized in that** the sterilization product is a fluid and **in that** its application (4) to the preform is effected by wetting at least the inner walls of the said preform (3) with a volume of fluid sufficient for the sterilization.

5. Process according to Claim 4, **characterized in that** the wetting (4) is effected by filling (41; ... 44) and then draining the preform, without drying the latter, so that a volume of fluid sufficient for the sterilization remains.

6. Process according to Claim 4, **characterized in that** the wetting (4) is effected by spraying (45, 46) into the preform the volume of fluid sufficient for the sterilization.

7. Process according to one of Claims 2 to 6, **characterized in that** the sterilization product is evaporable and the volume of sterilization product applied is such that it is completely evaporated after the drying before moulding.

8. Process according to one of Claims 2 to 7, **characterized in that** the sterilization product is an oxidizing agent.

9. Process according to Claim 8, **characterized in that** the sterilization product is based on hydrogen peroxide.

10. Process according to Claim 8, **characterized in that** the sterilization product is based on peracetic acid.

11. Process according to one of Claims 4 to 10, **characterized in that** the volume of fluid is less than one hundredth of the total volume of the preform.

12. Process according to Claim 11, **characterized in that** the volume of fluid is between five hundredths and three thousandths of the total volume of the preform.

13. Process according to one of Claims 1 to 12, **characterized in that** the blow moulding of the container (110) is effected with the aid of a sterile fluid, such as sterile air, so that the container can be immediately filled and/or stoppered after blow moulding.

14. Process according to one of Claims 1 to 12, **characterized in that** the blow moulding of the container is effected with the aid of filtered air and is completed, as a precaution, by an operation (114) of eliminating and/or destroying any sceptic particles reintroduced during blow moulding, so as to allow a subsequent possible filling and/or stoppering.

15. Process according to Claim 14, **characterized in that** the operation (114) of eliminating and/or destroying any sceptic particles reintroduced during blow moulding consists in rinsing the container with the aid of an appropriate sterilizing product, such as a solution of a sterilizing liquid, and in draining the container.

16. Process according to Claim 15, **characterized in that** the draining is followed by drying of the container.

17. Process according to one of Claims 1 to 16, **characterized in that** the preform is heated in two steps, a first step (115) for activating the action of the sterilization product and starting the rise in temperature of the preform, and a second step (100) for continuing the action of the sterilization product, evaporating it and giving the preform a defined heating profile for the purpose of moulding it.

18. Plant for implementing the process according to one of Claims 1 to 17, comprising means (100, 115) for heating the preforms and moulding means (101; 109) for blow moulding, or alternatively stretch-blow moulding, containers (110) from the heated preforms (3), and comprising, upstream of the heating means (100), means (4; 41 ... 44; 45) for applying the heat-activatable sterilization product to the said preforms (3) and means for transferring the preforms with the sterilization product into the heating means so as to activate the sterilization product and raise the preforms to their moulding temperature.

19. Plant according to Claim 18, **characterized in that** the sterilization product is a liquid and **in that** the means for applying the sterilization product to the preforms are designed to wet the preforms.

20. Plant according to Claim 19, **characterized in that** the means for wetting the preforms consist, on the one hand, of a series of nozzles (41; ...; 44) located above the path of the preforms upstream of the plant, so as to fill the preforms when they pass in line in front of these nozzles, and, on the other hand, by means (5) for inverting and emptying the preforms without drying them.

21. Plant according to Claim 20, **characterized in that** the means for inverting the preforms consist of inverting means (5) so as to take the preforms neck-down into the heating means (100).

22. Plant according to Claim 19, **characterized in that** the means for wetting the preforms consist of a sprayer (45), such as an electrically controlled automatic low-pressure spray gun.

23. Plant according to Claim 22, **characterized in that** the spray gun is supplied with air (451) and with spray liquid (450) and passage of the liquid is controlled by a control member (9) activated by a preform position sensor (10).

24. Plant according to one of Claims 18 to 23, **characterized in that** the heating means (100) for activating the sterilization product and raising the preforms to their moulding temperature consist of the oven (100) for thermally conditioning the preforms for the purpose of moulding them.

25. Plant according to Claims 18 to 23, **characterized in that** the heating means consist, on the one hand, of a heating chamber (115) downstream of the means (4) for applying the sterilization product and, on the other hand, of the oven (100) for heating the preforms in order to thermally condition them for the purpose of moulding the final containers (110), the said oven (100) is downstream of the chamber (115), and transfer means are provided between the chamber (115) and the oven (100) and are designed so that the preforms are not cooled during passage from the chamber to the oven.

26. Plant according to one of Claims 18 to 25, **characterized in that** it includes means for blowing sterile air into the preforms for the purpose of moulding them into final containers (110).

27. Plant according to one of Claims 18 to 25, **characterized in that** it includes means for blowing filtered air into the preforms for the purpose of moulding them into final containers (110).

28. Plant according to Claim 27, **characterized in that** it includes means (114) for carrying out an operation of eliminating and/or destroying any sceptic particles reintroduced during blow moulding, such as a rinsing and draining operation carried out on the containers (110).

29. Plant according to one of Claims 18 to 28, **characterized in that** it includes, upstream of the means (4; 41 ... 44; 45) for applying the sterilization product, means (49) for eliminating the static electricity present in the preforms.

30. Plant according to Claim 29, **characterized in that** the means for eliminating the static electricity consist of a device (49) for injecting ionized air into the preforms.

31. Plant according to one of Claims 18 to 30, **characterized in that** it is connected to a plant for filling and/or stoppering the containers (110).
